# EUROPEAN PATENT SPECIFICATION

(11) **EP 4 221 795 B1**
(45) Date of publication and mention of the grant of the patent: **13.12.2023**
(21) Application number: 21778096.4
(22) Date of filing: 17.09.2021
(51) Int. Cl.: A61M 16/06, A44B 11/00

(54) **SOFT SLIDE-THROUGH MECHANISM FOR CPAP HEADGEAR**
WEICHER DURCHSCHIEBEMECHANISMUS FÜR CPAP-KOPFBEDECKUNG
MÉCANISME DE FERMETURE À GLISSIÈRE SOUPLE POUR ENSEMBLE DE FIXATION DE MASQUE DE THÉRAPIE CPAP

(30) Priority: 30.09.2020 US 202063085374 P
(43) Date of publication of application: 09.08.2023
(73) Proprietor: Koninklijke Philips N.V., 5656 AG Eindhoven (NL)
(72) Inventor: STURM, Jordan, 5656 AE Eindhoven (NL); CHODKOWSKI, Lauren Patricia, 5656 AE Eindhoven (NL)
(74) Representative: Philips Intellectual Property & Standards
(86) International application number: PCT/EP2021/075588
(87) International publication number: WO 2022/069255

(56) References cited:
- EP-A1- 3 122 408
- WO-A1-2016/139623
- WO-A1-2017/042717
- US-A1- 2013 133 646

## Description

### CROSS-REFERENCE TO RELATED APPLICATIONS

This patent application claims the priority benefit under 35 U.S.C. § 119(e) of U.S. Provisional Application No. 63/085,374, filed on September 30, 2020.

### BACKGROUND OF THE INVENTION

### 1. Field of the Invention

The disclosed concept pertains to a headgear for use with a patient interface for supplying a pressurized flow of breathable gas to an airway of a patient. In particular, the present invention pertains to headgear that reduces tension in the neck of a patient.

### 2. Description of the Related Art

Patient interfaces are used to deliver a flow of breathing gas to a user in a variety of contexts. Pressurized breathing gas in particular is often used to treat medical disorders. For example, it is known to deliver positive airway pressure (PAP) to treat conditions such as chronic obstructive pulmonary disease (COPD) or sleep apnea syndrome, in particular, obstructive sleep apnea (OSA). Known PAP therapies include continuous positive airway pressure (CPAP), wherein a constant positive pressure is provided to the airway of the patient in order to splint open the patient's airway, and variable airway pressure, wherein the pressure provided to the airway of the patient is varied with the patient's respiratory cycle.

OSA is usually caused by an obstruction of the upper airway. It is characterized by repetitive pauses in breathing during sleep and is usually associated with a reduction in blood oxygen saturation. Non-invasive ventilation and pressure support therapies involve the placement of a patient interface device including a mask component on the face of a patient. The mask component may be, without limitation, a nasal mask that covers the patient's nose, a nasal cushion having nasal prongs that are received within the patient's nares, a nasal/oral mask that covers the nose and mouth, or a full face mask that covers the patient's face. The patient interface device interfaces a ventilator or pressure/flow generating device with the airway of the patient, so that a flow of breathing gas can be delivered from the ventilator or pressure/flow generating device to the airway of the patient. It is known to maintain such devices on the face of a wearer by a headgear having one or more straps adapted to fit over/around the patient's head. Because such patient interface devices are typically worn for an extended period of time, for example, overnight as a patient sleeps, it is important for the headgear to maintain the mask component of the device in a tight enough seal against the patient's face without discomfort.

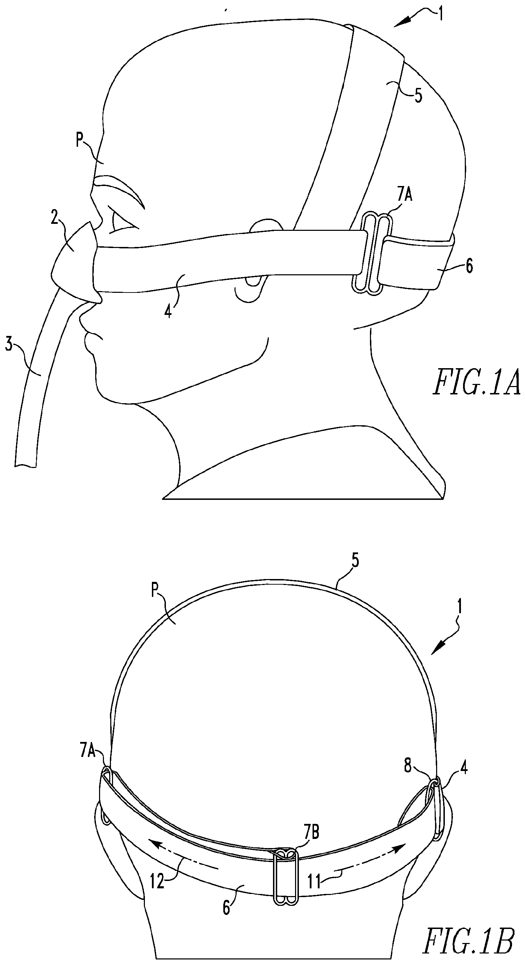

FIG. 1A is a side view and FIG. 1B is a rear view of a headgear 1 positioned on the head of a patient P. Headgear 1 includes a fit adjustment mechanism representative of fit adjustment mechanisms known in the relevant field, as is described in more detail herein. Headgear 1 secures a patient interface 2 to the head of patient P so that a pressurized flow of breathable gas generated by a respiratory therapy device (not shown), for example a CPAP machine, can be delivered to the airway of patient P via a delivery conduit 3 operatively coupled to patient interface 2. Headgear 1 includes a pair of side straps 4, a top strap 5 and a rear strap 6. To optimize the efficacy of the respiratory therapy, rear strap 6 must be taut enough to create a tight seal between patient interface 2 and the face of patient P in order to prevent the flow of pressurized gas from leaking out of patient interface 2.

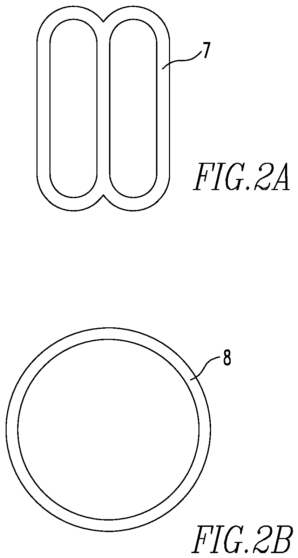

A pair of slide adjusters 7A, 7B (collectively, slide adjusters 7) and a ring 8, commonly used to adjust the length of several types of straps such as purse straps and camera straps, are used to adjust the fit of rear strap 6 on the head of patient P so that a sufficiently tight seal is achieved between patient interface 2 and the face of patient P. FIGS. 2A and 2B show a slide adjuster 7 and ring 8 as standalone components. Rear strap 6 is secured to the slide adjusters 7 and ring 8 as shown such that pulling rear strap 6 in the direction indicated by arrow 11 when slide adjuster 7B is held fixed in place loosens the fit of rear strap 6 around the head of patient P and pulling rear strap 6 in the direction indicated by arrow 12 when slide adjuster 7B is held fixed in place tightens the fit of rear strap 6 around the head of patient P.

Slide adjuster 7 and ring 8 are representative of various types of hardware that are known in the relevant field for adjusting the fit of headgear straps such as rear straps 6. Because slide adjuster 7, ring 8, and the various other types of hardware known and used in the relevant field for headgear strap length adjustment are usually produced from hard plastic or other similarly durable material, such hardware can create uncomfortable pressure points on a patient's head while the patient is sleeping. In addition to creating uncomfortable pressure points, such hardware can often be difficult to adjust, especially for older patients with dexterity issues.

The international patent application WO2016/0139623 discloses a respiratory mask system including a headgear that, in use, secures the respiratory mask system to a patient's head. The headgear includes a pair of forehead straps that are coupled together by a forehead coupler to form a closed loop. The forehead coupler is removably connected to a frame of the respiratory mask such that the forehead straps remain in a closed loop. The pair of forehead straps can also be coupled together by positioning a male strap portion within an aperture of a female strap portion. The male strap portion has a free end that is configured to be received into the aperture. The male strap portion includes a plurality of notches that engage the aperture of the female strap portion and provide incremental adjustment. The free ends of the male and female strap portions have fasteners configured to engage the surface of the other strap portion.

Common forms of headgear adjustments involve hard plastic pieces that can create uncomfortable pressure points on a patient's head. Elastic headgear without adjustability can achieve the same level of desired comfort, however, stretching out over time sacrifices an appropriate seal between the patient interface and the face of the patient. There is thus room for improvement in the design of headgear used to secure patient interfaces to the face of a patient.

### SUMMARY OF THE INVENTION

Accordingly, it is an object of the present invention to provide, in one embodiment, a headgear for use with a patient interface for supplying a pressurized flow of breathable gas to an airway of a patient, wherein the headgear is configured to secure the patient interface to a face of the patient and comprises a main body, the main body comprising: an interface pocket disposed between a first end of the main body and a second end of the main body opposite the first end, the pocket being configured to hold the patient interface and secure the patient interface against the face of the patient; a first lateral strap portion extending from a first edge of the interface pocket to the first end of the main body and configured to be disposed against a first side of the head of the patient, the first lateral strap portion comprising a first attachment portion and a slot; and a second lateral strap portion extending from a second edge of the interface pocket disposed opposite the first edge to the second end of the main body and configured to be disposed against a second side of the head of the patient, the second lateral strap portion being structured to pass through the slot in the first lateral strap portion and comprising a second attachment portion, wherein the first end of the main body is structured to be coupled to the second attachment portion and the second end of the main body is structured to be coupled to the first attachment portion.

In another embodiment, an arrangement for providing a flow of breathing gas to an airway of a patient comprises: a patient interface structured to be operatively coupled to a breathing gas generator via a delivery conduit and a headgear, wherein the headgear is configured to secure the patient interface to a face of the patient and comprises a main body, the main body comprising: an interface pocket disposed between a first end of the main body and a second end of the main body opposite the first end, the pocket being configured to hold the patient interface and secure the patient interface against the face of the patient; a first lateral strap portion extending from a first edge of the interface pocket to the first end of the main body and configured to be disposed against a first side of the head of the patient, the first lateral strap portion comprising a first attachment portion and a slot; and a second lateral strap portion extending from a second edge of the interface pocket disposed opposite the first edge to the second end of the main body and configured to be disposed against a second side of the head of the patient, the second lateral strap portion being structured to pass through the slot in the first lateral strap portion and comprising a second attachment portion, wherein the first end of the main body is structured to be coupled to the second attachment portion and the second end of the main body is structured to be coupled to the first attachment portion.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1A is a side view of a headgear with a fit adjustment mechanism representative of fit adjustment mechanisms known in the relevant field and shown positioned on the head of a patient;
FIG. 1B is a rear view of the headgear shown in FIG. 1A positioned on the head of the patient;
FIGS. 2A and 2B show separated hardware components from the headgear shown in FIGS. 1A and 1B;
FIG. 3A is a partial isometric view of a headgear in accordance with an exemplary embodiment of the present invention;
FIG. 3B is an alternative partial isometric view of the headgear shown in FIG. 3A in accordance with an exemplary embodiment of the present invention;
FIG. 4A shows an alternative mechanism for adjusting the straps of the headgear shown in FIGS. 3A and 3B in accordance with another exemplary embodiment of the present invention; and
FIG. 4B is a partial isometric view of a headgear that incorporates the mechanism shown in FIG. 4A in accordance with an exemplary embodiment of the present invention.

### DETAILED DESCRIPTION OF EXEMPLARY EMBODIMENTS

As used herein, the singular form of "a", "an", and "the" include plural references unless the context clearly dictates otherwise.

As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other. As used herein, "movably coupled" means that two components are coupled so as to allow at least one of the components to move in a manner such that the orientation of the at least one component relative to the other component changes.

As used herein, the statement that two or more parts or components are "integrated" shall mean that the parts or components are produced separately and subsequently joined together to produce a larger body. As used herein, the term "number" shall mean one or an integer greater than one (i.e., a plurality).

As used herein, the word "unitary" means a component is created as a single piece or unit. That is, a component that includes pieces that are created separately and then coupled together as a unit is not a "unitary" component or body.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

The disclosed concept, as described in greater detail herein in connection with various particular exemplary embodiments, pertains to improvements in headgear for use with patient interfaces for supplying a pressurized flow of breathable gas to an airway of a patient, and specifically to improvements in mechanisms for tightening the fit of such headgear on the head of a patient.

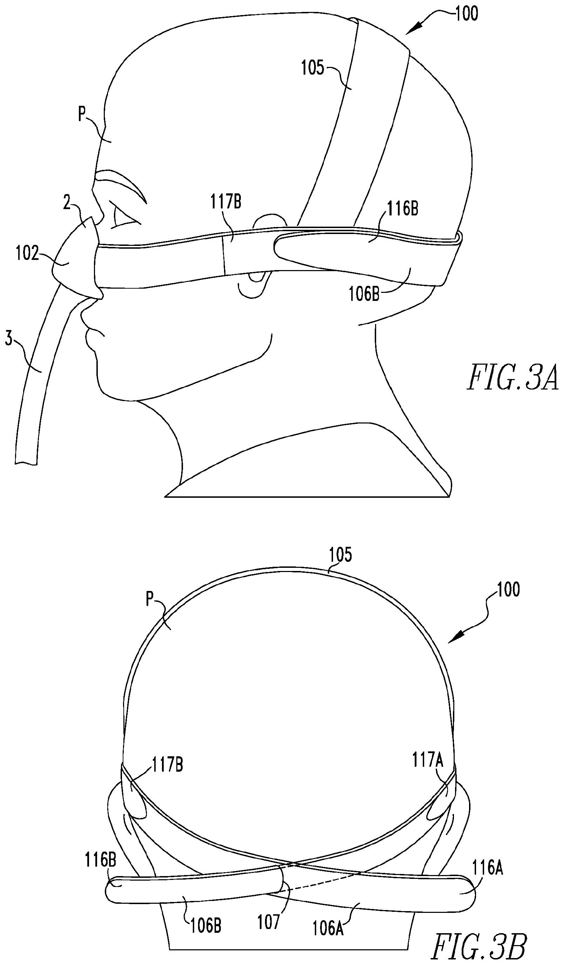

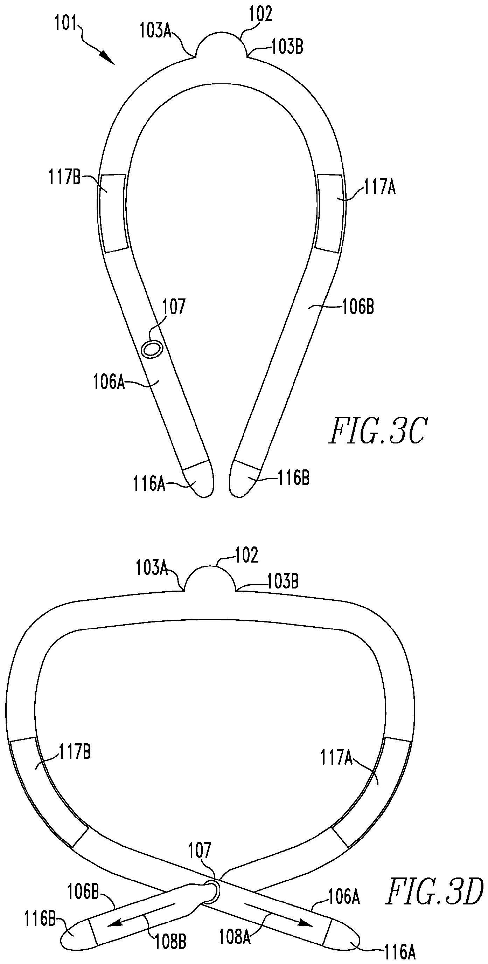

FIG. 3A is a side view and FIG. 3B is a rear view of a headgear 100 that includes a fit adjustment mechanism in accordance with a non-limiting exemplary embodiment of the disclosed concept positioned on the head of patient P. FIGS. 3C and 3D show partial overhead isometric views of headgear 100 when not positioned on the head of patient P. Headgear 100 secures a patient interface 2 to the head of patient P so that a pressurized flow of breathable gas generated by a respiratory therapy device (not shown) can be delivered to the airway of patient P via a delivery conduit 3 operatively coupled to patient interface 2. A main body 101 (as shown in FIG. 3C) of headgear 100 is constructed by sewing a tube of fabric with a pocket 102 disposed approximately halfway between the two ends of the tube in which patient interface 2 can sit. It will be appreciated that in the view shown in FIG. 3A, the surface of patient interface 2 is covered by the fabric of pocket 102.

While patient interface 2 is depicted as being a nasal interface in FIG. 3A, it will be appreciated that headgear 100 could be constructed from a tube of fabric with a pocket sufficiently sized to accommodate other patient interfaces with differing dimensions and shapes, such as a patient interface covering both the nose and mouth, without departing from the scope of the disclosed concept. It will be further appreciated that headgear 100 could be constructed from material other than fabric, for example and without limitation neoprene or other soft polymers, without departing from the scope of the disclosed concept.

In addition to main body 101, headgear 100 includes a top strap 105 that is coupled to main body 101 and assists in keeping headgear 100 from slipping down (relative to the views of FIGS. 3A and 3B) the head of patient P. While top strap 105 is depicted as a single strap crossing over the head of patient P from a region near one ear of patient P to the other ear of patient P, it will be appreciated that top strap 105 is provided as a non-limiting example of additional support that can be included in headgear 100, and that substitutes for top strap 105 may be used or top strap 105 may be omitted without departing from the scope of the disclosed concept.

To optimize the efficacy of the respiratory therapy, lateral straps 106A, 106B (collectively, lateral straps 106) of main body 101 must be taut enough to create a tight seal between patient interface 2 and the face of patient P in order to prevent the flow of pressurized gas from leaking out of patient interface 2. Lateral strap 106A commences at an edge 103A of pocket 102 and extends to an end 116A of main body 101, and lateral strap 106B commences at an edge 103B of pocket 102 and extends to an end 116B of main body 101. Lateral strap 106A includes a slot 107 into which lateral strap 106B can be inserted and pass through. Slot 107 is produced by creating an opening in lateral strap 106A, and it will be appreciated that slot 107 could be reinforced with foam or other similar material to give added structure if necessary. Because headgear 100 is produced from fabric or other flexible material, lateral strap 106B can compress and/or conform to a shape that allows lateral strap 106B to be inserted into and pass through slot 107 easily.

Referring to FIG. 3D, after lateral strap 106B has been inserted into slot 107 as shown, the fit of headgear 100 on the head of patient P can be adjusted by pulling ends 116A, 116B of lateral straps 106 away from slot 107 in the general directions indicated by arrows 108A, 108B (shown in FIG. 3D) and attaching ends 116A, 116B of lateral straps 106A, 106B to their corresponding attachment points 117A, 117B located on lateral straps 106B, 106A respectively. It will be appreciated that the fabric of headgear 100 can be sewn in such a way that it does not bunch and create a build-up of material on the back of the head of patient P when lateral straps 106A, 106B are pulled to tighten the fit of headgear 100 around the head of patient P. In an exemplary embodiment, attachment points 117A, 117B comprise the hook portion of a hook and loop fastener and ends 116A, 116B comprise the loop portion of a hook and loop fastener.

Using slot 107 to adjust the fit of headgear 100 and using hook and loop fasteners to couple ends 116A, 116B to attachment points 117A, 117B eliminates the uncomfortable pressure points created by hardware such as slide adjusters 7, ring 8, and other hardware known in the relevant field. In addition, being able to adjust the seal of patient interface 2 against the face by simply pulling lateral straps 106 and to secure lateral straps 106 with hook and loop fasteners is more accessible for patients with dexterity issues than making adjustments using fine motor skills as hardware typically used in the relevant field requires. While FIG. 3A depicts only lateral strap 106A as having a slot 107, it will be appreciated that lateral strap 106B could include slot 107 instead of lateral strap 106A such that lateral strap 106A would be inserted through slot 107, or that both rear straps 106 could include a slot 107 such that either lateral strap 106 could be inserted through the slot 107 of the other lateral strap 106, without departing from the scope of the disclosed concept. In addition, to the extent that securing mechanisms other than hook and loop fasteners can secure lateral straps 106 without creating pressure points on the head of patient and without creating difficulties for patients with dexterity issues, such securing mechanisms can be used without departing from the scope of the disclosed concept.

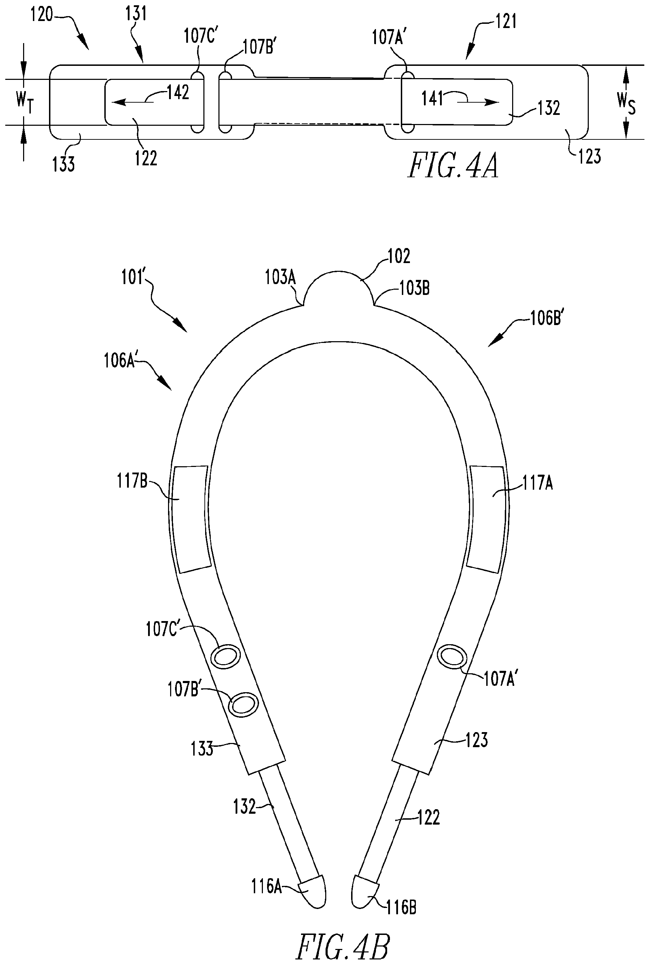

FIG. 4A shows a slide-through mechanism 120 that can be used for strap tautness adjustment, and FIG. 4B shows a main body 101' into which slide-through mechanism 120 is incorporated, in accordance with another non-limiting exemplary embodiment of the disclosed concept. Main body 101' is substantially similar to main body 101, comprising several of the same components as main body 101 and operating in substantially the same manner as main body 101. It will be appreciated that elements of main body 101 and main body 101' that are identified with the same reference numbers in FIGS. 3A-3D and 4A-4B are the same elements and operate in the same manner in the different embodiments. With respect to main body 101', instead of comprising lateral straps 106 and including slot 107 from main body 101, main body 101' comprises lateral straps 106A', 106B' (collectively, lateral straps 106') which incorporate slots 107A', 107B', 107C' (collectively, slots 107') from slide-through mechanism 120 shown in FIG. 4A. Main body 101' is constructed in the same manner as main body 101, and slots 107' are produced in the same manner as slot 107, i.e., by creating openings in lateral straps 106'. As with slot 107, slots 107' could be reinforced with foam or other similar material to give added structure if necessary. In addition, main body 101' could be coupled to a top such as top strap 105 from FIGS. 3A and 3B. It will be appreciated that top strap 105 is provided as a non-limiting example of additional support that can be coupled to main body 101', and that substitutes for top strap 105 may be used or that top strap 105 may be omitted without departing from the scope of the disclosed concept.

Referring to FIG. 4A, slide-through mechanism 120 comprises two sliding members, a single slot member 121 and a double slot member 131. As previously stated, the design of slide-through mechanism 120 is incorporated into main body 101', and single slot member 121 should be considered representative of one of either lateral strap 106A' or lateral strap 106B' while double slot member 131 should be considered representative of the other of lateral strap 106A' or lateral strap 106B'. A first end of single slot member 121 comprises a tab 122 of width w_{T} and a second end disposed opposite of the first end comprises a stop 123 of width ws. Similarly, a first end of double slot member 131 comprises a tab 132 of width w_{T} and a second end disposed opposite of the first end comprises a stop 133 of width ws. Slot 107A' of single slot member 121 is constructed to be wide enough to allow tab 132 to pass through slot 107A' when tab 132 is pulled as indicated by arrow 141, and slots 107B', 107C' are similarly constructed to be wide enough to allow tab 131 to pass through slots 107B', 107C' when tab 131 is pulled as indicated by arrow 142. Pulling tabs 122, 132 in the directions indicated by arrows 141, 142 tightens the fit of main body 101' against the head of patient P.

In FIG. 4A, tabs 122 and 132 are depicted as being of the same width w_{T} and stops 123 and 133 are depicted as being of the same width ws, with width ws being greater than width w_{T}. The difference in the widths of tabs 122, 132 and stops 123, 133 creates a limit on how far tab 122 can be pulled in the direction indicated by arrow 142 and on how far tab 132 can be pulled in the direction indicated by arrow 141, which is useful for preventing overtightening of main body 101' as well as for controlling alignment zones for the hook and loop fasteners used to secure ends 116A', 116B' to attachment points 117A, 117B. It will be appreciated, however, that the relative dimensions and scale of the components of slide mechanism 120 shown in FIG. 4A are intended to be representative and that the widths of tabs 131 and 132 relative to one another, the widths of stops 123 and 133 relative to one another, and the widths of the tabs 131, 132 relative to the stops 123, 133 can all deviate from the proportions shown in FIG. 4A without departing from the scope of the disclosed concept. In addition, if preventing overtightening of main body 101' and aligning ends 116A, 116B with attachment points 117A, 117B are not a priority, slides 121 and 131 can be produced to be of uniform width (as opposed to being produced to comprise tabs and stops, with the tabs being of lesser width than the stops) without departing from the scope of the disclosed concept. Main body 101', like main body 101, is produced from fabric or other flexible material, and as such, it will be appreciated that slides 121 and 131 could be produced with the capability to compress or otherwise contort in order to be inserted into and pulled through slots 107' if slides 121 and 131 are produced to be of uniform width.

Similar to main body 101, incorporating the design of slide-through mechanism 120 into main body 101' eliminates the need for hardware such as slide adjuster 1 and ring 2 or other hardware known in the relevant field, thereby eliminating any pressure points on the head of a patient created by such hardware. In addition, being able to adjust the seal of patient interface 2 against the face by simply pulling lateral straps 106' and to secure lateral straps 106' with hook and loop fasteners is more accessible for patients with dexterity issues than making adjustments using fine motor skills as hardware typically used in the relevant field requires.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims.

The invention is defined by the appended claims. Subject-matter referred as embodiments and/or disclosures which are not claimed are not part of the invention.

## Claims

1. A headgear (100) for use with a patient interface for supplying a pressurized flow of breathable gas to an airway of a patient, wherein the headgear is configured to secure the patient interface to a face of the patient, the headgear comprising:
a main body (101, 101'), the main body comprising:
an interface pocket (102) disposed between a first end of the main body and a second end of the main body opposite the first end, wherein the pocket being configured to hold the patient interface and secure the patient interface against the face of the patient; and in that the main body also comprises:
a first lateral strap portion (106A, 106B') extending from a first edge (103A, 103B) of the interface pocket to the first end of the main body and configured to be disposed against a first side of the head of the patient, the first lateral strap portion comprising a first attachment portion (117B, 117A) and a slot (107, 107A'); and
a second lateral strap portion (106B, 106A') extending from a second edge of the interface pocket disposed opposite the first edge to the second end of the main body and configured to be disposed against a second side of the head of the patient, the second lateral strap portion being structured to pass through the slot in the first lateral strap portion and comprising a second attachment portion (117A, 117B),
wherein the first end of the main body is structured to be coupled to the second attachment portion and the second end of the main body is structured to be coupled to the first attachment portion.

2. The headgear of claim 1, further comprising a top strap (105) configured to be disposed on a top side of a head of the patient and coupled to the first lateral strap portion at a first end of the top strap and coupled to the second lateral strap portion at a second end of the top strap disposed opposite the first end.

3. The headgear of claim 1, wherein the main body is produced from fabric.

4. The headgear of claim 1, wherein the main body is produced from a number of soft polymers.

5. The headgear of claim 1, wherein the first end of the main body and the second end of the main body each comprise one of a hook or a loop of a hook and loop fastener, and wherein the first attachment portion and second attachment portion each comprise the other of a hook or a loop of a hook and loop fastener.

6. The headgear of claim 1, wherein the second lateral strap portion comprises two slots, and wherein the first lateral strap portion is structured to pass through the two slots in the second lateral strap portion.

7. The headgear of claim 6:
wherein the first lateral strap portion (106B') and the second lateral strap portion each comprise a tab section and a stop section,
wherein the stop section (123) of the first lateral strap portion is wider than the tab section of the first lateral strap portion and the two slots of the second lateral strap portion, and
wherein the stop section (133) of the second lateral strap portion is wider than the tab section of the second lateral strap portion and the slot of the first lateral strap portion.

8. The headgear of claim 7:
wherein the stop section of the first lateral strap portion cannot pass through the two slots of the second lateral strap portion, and
wherein the stop section of the second lateral strap portion cannot pass through the slot of the first lateral strap portion.

9. An arrangement for providing a flow of breathing gas to an airway of a patient, the arrangement comprising:
a patient interface (2) structured to be operatively coupled to a breathing gas generator via a delivery conduit; and
a headgear (100) as claimed in claim 1.

## Patentansprüche

1. Kopfbedeckung (100) zur Verwendung mit einer Patientenschnittstelle zum Zuführen eines unter Druck stehenden Atemgasstroms zu den Atemwegen eines Patienten, wobei die Kopfbedeckung konfiguriert ist, um die Patientenschnittstelle an einem Gesicht des Patienten zu befestigen, wobei die Kopfbedeckung Folgendes umfasst:
einen Hauptkörper (101, 101'), wobei der Hauptkörper umfasst:
eine Schnittstellentasche (102), die zwischen einem ersten Ende des Hauptkörpers und einem zweiten Ende des Hauptkörpers gegenüber dem ersten Ende angeordnet ist, wobei die Tasche konfiguriert ist, um die Patientenschnittstelle zu halten und die Patientenschnittstelle am Gesicht des Patienten zu befestigen; und dass der Hauptkörper außerdem Folgendes umfasst:
einen ersten seitlichen Riemenabschnitt (106A, 106B'), der sich von einer ersten Kante (103A, 103B) der Schnittstellentasche zum ersten Ende des Hauptkörpers erstreckt, und so konfiguriert ist, dass er an einer ersten Seite des Kopfes des Patienten angelegt werden kann, wobei der erste seitliche Riemenabschnitt einen ersten Befestigungsabschnitt (117B, 117A) und einen Schlitz (107, 107A') umfasst; und
einen zweiten seitlichen Riemenabschnitt (106B, 106A'), der sich von einer zweiten Kante der Schnittstellentasche, die gegenüber der ersten Kante angeordnet ist, bis zum zweiten Ende des Hauptkörpers erstreckt, und so konfiguriert ist, dass er an einer zweiten Seite des Kopfes des Patienten angebracht werden kann, wobei der zweite seitliche Riemenabschnitt so strukturiert ist, dass er durch den Schlitz im ersten seitlichen Riemenabschnitt verläuft, und einen zweiten Befestigungsabschnitt (117A, 117B) umfasst, wobei das erste Ende des Hauptkörpers so strukturiert ist, dass es mit dem zweiten Befestigungsabschnitt gekoppelt werden kann, und das zweite Ende des Hauptkörpers so strukturiert ist, dass es mit dem ersten Befestigungsabschnitt gekoppelt werden kann.

2. Kopfbedeckung nach Anspruch 1, ferner umfassend einen oberen Riemen (105), der so konfiguriert ist, dass er auf einer Oberseite eines Kopfes des Patienten angeordnet ist, und an einem ersten Ende des oberen Riemens mit dem ersten seitlichen Riemenabschnitt verbunden ist, und mit diesem verbunden werden kann, und an einem zweiten Ende des oberen Riemens, das gegenüber dem ersten Ende angeordnet ist, mit dem zweiten seitlichen Riemenabschnitt verbunden ist.

3. Kopfbedeckung nach Anspruch 1, wobei der Hauptkörper aus Stoff hergestellt ist.

4. Kopfbedeckung nach Anspruch 1, wobei der Hauptkörper aus mehreren weichen Polymeren hergestellt ist.

5. Kopfbedeckung nach Anspruch 1, wobei das erste Ende des Hauptkörpers und das zweite Ende des Hauptkörpers jeweils einen von einem Haken oder einer Schlaufe eines Klettverschlusses umfassen, und wobei der erste Befestigungsabschnitt und der zweite Befestigungsabschnitt jeweils den anderen aus einem Haken oder einer Schlaufe eines Klettverschlusses umfassen.

6. Kopfbedeckung nach Anspruch 1, wobei der zweite seitliche Riemenabschnitt zwei Schlitze umfasst, und wobei der erste seitliche Riemenabschnitt so strukturiert ist, dass er durch die beiden Schlitze im zweiten seitlichen Riemenabschnitt verläuft.

7. Kopfbedeckung nach Anspruch 6:
wobei der erste seitliche Riemenabschnitt (106B') und der zweite seitliche Riemenabschnitt jeweils einen Laschenabschnitt und einen Anschlagabschnitt umfassen,
wobei der Anschlagabschnitt (123) des ersten seitlichen Riemenabschnitts breiter ist als der Laschenabschnitt des ersten seitlichen Riemenabschnitts und die beiden Schlitze des zweiten seitlichen Riemenabschnitts, und
wobei der Anschlagabschnitt (133) des zweiten seitlichen Riemenabschnitts breiter ist als der Laschenabschnitt des zweiten seitlichen Riemenabschnitts und der Schlitz des ersten seitlichen Riemenabschnitts.

8. Kopfbedeckung nach Anspruch 7:
wobei der Anschlagabschnitt des ersten seitlichen Riemenabschnitts nicht durch die beiden Schlitze des zweiten seitlichen Riemenabschnitts hindurchgehen kann, und
wobei der Anschlagabschnitt des zweiten seitlichen Riemenabschnitts nicht durch den Schlitz des ersten seitlichen Riemenabschnitts hindurchgehen kann.

9. Anordnung zum Bereitstellen eines Atemgasstroms für die Atemwege eines Patienten, wobei die Anordnung Folgendes umfasst:
eine Patientenschnittstelle (2), die so strukturiert ist, dass sie über eine Zufuhrleitung operativ mit einem Atemgasgenerator verbunden werden kann; und
eine Kopfbedeckung (100) nach Anspruch 1.

## Revendications

1. Harnais de tête (100) destiné à être utilisé avec une interface patient pour fournir un flux sous pression de gaz respirable aux voies respiratoires d'un patient, où le harnais de tête est configuré pour maintenir l'interface patient sur le visage du patient, le harnais de tête comprenant:
un corps principal (101, 101'), le corps principal comprenant:
une poche d'interface (102) disposée entre une première extrémité du corps principal et une seconde extrémité du corps principal opposée à la première extrémité, où la poche est configurée pour tenir l'interface patient et maintenir l'interface patient contre le visage du patient;
et en ce que le corps principal comprend également :
une première partie de sangle latérale (106A, 106B') s'étendant depuis un premier bord (103A, 103B) de la poche d'interface jusqu'à la première extrémité du corps principal et étant configurée pour être disposée contre un premier côté de la tête du patient, la première partie de sangle latérale comprenant une première partie de fixation (117B, 117A) et une fente (107, 107A'); et
une seconde partie de sangle latérale (106B, 106A') s'étendant depuis un second bord de la poche d'interface disposé opposé au premier bord jusqu'à la seconde extrémité du corps principal et étant configurée pour être disposée contre un second côté de la tête du patient, la seconde partie de sangle latérale étant structurée pour passer à travers la fente dans la première partie de sangle latérale et comprenant une seconde partie de fixation (117A, 117B),
où la première extrémité du corps principal est structurée pour être couplée à la seconde partie de fixation et la seconde extrémité du corps principal est structurée pour être couplée à la première partie de fixation.

2. Harnais de tête selon la revendication 1, comprenant en outre une sangle supérieure (105) configurée pour être disposée sur un côté supérieur de la tête du patient et couplée à la première partie de sangle latérale au niveau d'une première extrémité de la sangle supérieure et couplée à la seconde partie de sangle latérale au niveau d'une seconde extrémité de la sangle supérieure disposée opposée à la première extrémité.

3. Harnais de tête selon la revendication 1, dans lequel le corps principal est réalisé à partir de tissu.

4. Harnais de tête selon la revendication 1, dans lequel le corps principal est réalisé à partir d'un certain nombre de polymères souples.

5. Harnais de tête selon la revendication 1, dans lequel la première extrémité du corps principal et la seconde extrémité du corps principal comprennent chacune l'une entre une partie à crochets ou une partie à boucles d'une fermeture autoagrippante, et dans lequel la première partie de fixation et la seconde partie de fixation comprennent chacune l'autre d'une partie à crochets ou une partie à boucles d'une fermeture auto-agrippante.

6. Harnais de tête selon la revendication 1, dans lequel la seconde partie de sangle latérale comprend deux fentes, et dans lequel la première partie de sangle latérale est structurée pour passer à travers les deux fentes dans la seconde partie de sangle latérale.

7. Harnais de tête selon la revendication 6:
dans lequel la première partie de sangle latérale (106B') et la seconde partie de sangle latérale comprennent chacune une section de languette et une section de arrêt,
dans lequel la section d'arrêt (123) de la première partie de sangle latérale est plus large que la section de languette de la première partie de sangle latérale et que les deux fentes de la seconde partie de sangle latérale, et
dans lequel la section d'arrêt (133) de la seconde partie de sangle latérale est plus large que la section de languette de la seconde partie de sangle latérale et que la fente de la première partie de sangle latérale.

8. Harnais de tête selon la revendication 7:
dans lequel la section d'arrêt de la première partie de sangle latérale ne peut pas traverser les deux fentes de la seconde partie de sangle latérale, et
dans lequel la section d'arrêt de la seconde partie de sangle latérale ne peut pas passer à travers la fente de la première partie de sangle latérale.

9. Agencement pour fournir un flux de gaz respiratoire aux voies respiratoires d'un patient, l'agencement comprenant:
une interface patient (2) structurée pour être fonctionnellement couplée à un générateur de gaz respiratoire via un conduit d'administration; et
un harnais de tête (100) tel que revendiqué dans la revendication 1.
